Europäisches Patentamt

European Patent Office

Office européen des brevets

(19)

(11) Veröffentlichungsnummer: **0 221 973**
**B1**

(12) ⌀ **EUROPÄISCHE PATENTSCHRIFT**

(45) Veröffentlichungstag der Patentschrift: **08.08.90**

(21) Anmeldenummer: **86903317.5**

(22) Anmeldetag: **10.05.86**

(86) Internationale Anmeldenummer:
**PCT/EP86/00274**

(87) Internationale Veröffentlichungsnummer:
**WO 86/06970 04.12.86 Gazette 86/26**

(51) Int. Cl.⁵: **A 61 M 16/20, A 61 M 16/04**

(54) **TRACHEOSTOMA-VERSCHLUSS.**

(30) Priorität: **21.05.85 DE 8514859 u**

(43) Veröffentlichungstag der Anmeldung:
**20.05.87 Patentblatt 87/21**

(45) Bekanntmachung des Hinweises auf die
Patenterteilung:
**08.08.90 Patentblatt 90/32**

(84) Benannte Vertragsstaaten:
**DE FR GB SE**

(56) Entgegenhaltungen:
**EP-A- 078 685
EP-A-0 132 957
CH-A- 408 292
US-A-3 721 233
US-A-3 952 335
US-A-4 040 428**

(73) Patentinhaber: **ESKA medical &
kunststofftechnik gmbh & co., vormals Walter
Koss
Industriestrasse 2 Postfach 1164
D-6222 Geisenheim am Rhein (DE)**

(72) Erfinder: **HERRMANN, Ingo, F.
Burkarderstrasse 34 c
D-8700 Würzburg (DE)**

(74) Vertreter: **Blumbach Weser Bergen Kramer
Zwirner Hoffmann Patentanwälte
Sonnenberger Strasse 100
D-6200 Wiesbaden (DE)**

Anmerkung: Innerhalb von neun Monaten nach der Bekanntmachung des Hinweises auf die Erteilung des europäischen
Patents im Europäischen Patentblatt kann jedermann beim Europäischen Patentamt gegen das erteilte europäische
Patent Einspruch einlegen. Der Einspruch ist schriftlich einzureichen und zu begründen. Er gilt erst als eingelegt,
wenn die Einspruchsgebühr entrichtet worden ist (Art. 99(1) Europäisches Patentübereinkommen).

**Beschreibung**

Die Erfindung betrifft einen Tracheostoma-Verschluß nach dem Oberbegriff des Anspruchs 1. Ein solcher Verschluß ist aus der US-A-3 952 335 bekannt. Ein ähnliches Tracheostoma-Ventil ist weiterhin aus der DE-U-83 29 888 bekannt.

Patienten mit einem Tracheostoma, d.h. einer künstlichen, im Halsbereich in den Luftweg führenden Öffnung, können nach Totaloperationen des Kehlkopfs in bekannter Weise mit Hilfe einer Sprech oder Stimm-Prothese sprechen, die als Verbindung zwischen dem Luftweg und dem Speiseweg operativ eingesetzt wird. Zum Sprechen muß dann aber das Tracheostoma verschlossen werden. Dazu benützen die Patienten üblicherweise einen Finger, der auf die Öffnung gelegt wird und das Ausströmen der Luft an dieser Stelle verhindert. Zur Vereinfachung des Sprechens ist es bereits bekannt, ein Membranventil auf der Öffnung anzubringen, und zwar unter Verwendung einer Klebefolie. Es treten dabei jedoch Schwierigkeiten hinsichtlich der Haltbarkeit und Abdichtung ein. Außerdem sind solche Ventile hinsichtlich ihrer Funktion empfindlich gegen Verschmutzungen.

Die aus den obengenannten Druckschriften bekannten Tracheostoma-Verschlüsse stellen eine bewährte Lösung für die erläuterten Probleme dar. Sie ermöglichen eine einfache und sichere Anbringung und bequeme Handhabung durch den Patienten. Es sind aber Verbesserungen erwünscht. Der Erfindung liegt demgemäß die Aufgabe zugrunde, die bekannten Tracheostoma-Verschlüsse so weiterzuentwickeln, daß ein größerer Luftdurchsatz bei geringerer Bauhöhe und weiter verbesserter Anbringung und Handhabung ermöglicht wird. Zwei Lösungen dieser Aufgabe sind in den Patentansprüchen 1 und 2 angegeben.

Im ersten Fall weist die Ventilkappe zur Vermeidung der Lageabhängigkeit des Schließpunktes, beispielsweise beim Bücken, ein magnetisch wirksames Bauteil auf, das mit einem weiteren magnetisch wirksamen Bauteil am oder in der Wand des Ventilgehäuses zusammenwirkt und die Ventilkappe im geöffneten Zustand hält. Dabei ist wenigstens eines der magnetisch wirksamen Bauteile ein Permanentmagnet. Das andere Bauteil kann aus weichmagnetischem Material, beispielsweise einem Eisenplättchen bestehen.

Im anderen Fall ist die Ventilklappe im in der Betriebslage unteren Bereich des Ventilgehäuses gelagert und wird durch ein Zusatzgewicht in der geöffneten Lage gehalten. Die Ventilklappe erfüllt ihre Rückschlagfunktion dann erst, wenn eine bestimmte Strömungsgeschwindigkeit beim Atmen überschritten wird, der Patient also willkürlich durch einen plötzlichen Atemstoß die Ventilklappe schließen will, um zu sprechen.

Es sind zahlreiche Weiterbildungen möglich. So kann die Ventilklappe und/oder ihr Sitz aus nachgiebigem Material bestehen, derart, daß die Klappe nach dem Schließen durch ihren Sitz hindurchtreten kann, wenn der vorgegebene Staudruck überschritten wird. Durch diese besondere Ausbildung wird für die notwendige Sicherheit gesorgt, denn ein plötzlicher Atemausstoß, beispielsweise beim Husten, muß immer möglich bleiben. Die Ventilklappe kann, wenn sie durch ihren Sitz hindurchgetreten ist, ohne Herausnehmen des Verschlusses von Hand wieder in ihre normale Lage zurückgedrückt werden.

Das Sicherheitsmerkmal kann aber auch in an sich bekannter Weise dadurch verwirklicht sein, daß das Ventilgehäuse auf den Kanülenstummel aufgesteckt ist und dann notfalls abgehustet oder abgezogen werden kann.

Eine weitere Möglichkeit, die Ventilklappe bis zu einer bestimmten Ausströmgeschwindigkeit im geöffneten Zustand zu halten, besteht darin, die Klappe durch Federkraft in die geöffnete Stellung zu drängen. Die Federkraft kann durch die Lagerung der Klappe selbst, beispielsweise in Form eines Filmscharniers, oder auch durch eine getrennte Feder aufgebracht werden.

Zur Anpassung an die jeweiligen Patienten und ihre Lebens- und Sprechgewohnheiten ist es vorteilhaft, wenn die Kraft, mit der die Ventilklappe im geöffneten Zustand gehalten wird, leicht einstellbar ist. Dazu sieht eine Weiterbildung der Erfindung vor, daß die Lagerstelle der Ventilklappe in Umfangsrichtung des Kanülenstummels verschiebbar ist. Im falle eines Zusatzgewichts kann dann die wirksame Schwerkraftkomponente verändert werden, und im Fall von Magneten verändert die Verschiebung der Lagerstelle die Magnetkraft durch Änderung der sich gegenüberliegenden wirksamen Flächen der Magneten. Es besteht aber auch die Möglichkeit, zur Einstellung der Ansprechempfindlichkeit den Abstand der Magnete im geöffneten Zustand der Ventilklappe einstellbar zu machen, beispielsweise mit Hilfe einer Anschlagschraube.

Zur Verwirklichung der Verschiebung in Umfangsrichtung kann vorgesehen sein, daß das Ventilgehäuse auf den Kanülenstummel aufgesteckt und gegen diesen verdrehbar ist. Um beim Verdrehen des Ventilgehäuses gegen den Kanülenstummel zwecks Einstellung der Ansprechempfindlichkeit die auf den Kanülenstummel und damit das Stoma und die Trachea ausgeübten kräfte gering zu halten, kann vorgesehen sein, daß der Kanülenstummel im Bereich seiner Berührungsfläche mit dem Ventilgehäuse einen umlaufenden Steg aufweist. Das Ventilgehäuse gleitet dann mit seinem Innenrand auf dem schmalen Steg statt auf der gesamten Berührungsfläche. Damit das Ventilgehäuse nicht zu leicht abfallen kann, kann im Bereich seiner Berührungsfläche mit dem Kanülenstummel ebenfalls ein umlaufender Steg angeordnet sein, der beim Aufdrücken des Ventilgehäuses auf den Kanülenstummel hinter dessen Steg schnappt. Einer der umlaufenden Stege kann auch ohne Beeinträchtigung der Dichtigkeit Unterbrechungen aufweisen.

Das Ventilgehäuse kann in Weiterbildung der Erfindung aber auch einstückig Teil des Kanülenstummels sein. Hierbei, aber auch bei getrenntem Ventilgehäuse kann zweckmäßig die Ventilklappe die Form einer dünnen Scheibe haben, die über ein Filmscharnier mit einem radialen Fortsatz verbunden ist, welcher abdichtend durch einen Schlitz in der Wand des Ventilgehäuses gesteckt ist und vorzugsweise einen Anschlag aufweist, der die Einstecktiefe definiert und begrenzt. Zur Erzielung der Einstellbarkeit hinsichtlich der zum Schließen erforderlichen Luftgeschwindigkeit bei einstückiger Ausbildung von Kanülenstummel und Ventilgehäuse kann der Schlitz in Umfangsrichtung des Ventilgehäuses so breit sein, daß der Fortsatz der Ventilklappe in seiner Winkellage verstellbar ist.

In einigen Fällen aht sich gezeigt, daß im unteren Bereich des Tracheostomas eine Einwölbung oder Einsenkung entsteht, die die Abdichtung erschwert. Hierzu sieht eine Weiterbildung der Erfindung vor, daß der in diesem Bereich anliegende, verschiebbare Flanschring auf einem Teil seines Umfangs eine Ausnehmung aufweist. Zusätzlich oder statt dessen kann der Flanschring auch aus unterschiedlich hartem Material hergestellt werden, derart, daß im Bereich der Einwölbung weiches, gut anliegendes Material vorhanden ist. Weiterhin ist eine individuelle Formanpassung des Flanschrings an die anatomischen Verhältnisse möglich. Zusätzlich kann der Flanschring mit einer eine Bohrung aufweisenden, radial abstehenden Lasche versehen sein, die die Anbringung eines zum abnehmbaren Ventilgehäuse führenden Bandes ermöglicht. Das Ventilgehäuse ist dann gegen Verlieren gesichert. Statt dessen kann das Ventilgehäuse mit einem Band auch an einer radial abstehenden Lasche des Kanülenstummels befestigt sein. Die Flanschringe lassen sich dann symmetrisch ausbilden, so daß sie vom Patienten leichter handhabbar sind.

Das Abschlußorgan besteht bei dem eingangs erläuterten, bekannten Tracheostoma-Verschluß aus einem Flansch. In Weiterbildung der Erfindung ist vorgesehen. daß das Abschlußorgan ein an das innere Ende des Kanülenstummels angesetzten, gegen den Kanülenstummel offenes Rohrstück ist. Das setzt voraus, daß die Trachea nicht in der sonst üblichen Weise unter Biegung zum Stoma geführt ist, sondern daß die Trachea nach Absetzen des Kehlkopfes einen Deckel erhält und das Stoma seitlich in die Trachea geführt ist. Dadurch wird auch ein großlumiges Stoma mit einem entsprechend großen Querschnitt des Verschlusses ermöglicht.

Die Enden des Rohrstücks werden zweckmäßig schräg abgesetzt, derart, daß das Rohrstück an der dem Kanülenstummel entgegengesetzten Seite kürzer ist. Zum Einsetzen einer Stimmprothese in den Tracheostoma-Verschluß weist das Rohrstück an der dem Kanülenstummel gegenüberliegenden Wand mit Vorteil eine Öffnung auf.

Die Stimmprothese kann dann durch diese Öffnung und die Wand der Trachea in die Speiseröhre führen. Eine andere Möglichkeit besteht darin, das Rohrstück auf der dem Kanülenstamm gegenüberliegenden Seite vollständig zu öffnen, so daß anstelle des Rohres ein rinnenförmiges Gebilde entsteht.

Der Tracheostoma-Verschluß nach der Erfindung wird zweckmäßig aus einem elastomeren Kunststoff, vorzugsweise Silicongummi, hergestellt. Das Rohrstück wird dabei zur Vermeidung von Irritationen im Bereich seiner Enden aus weich eingestelltem Material gefertigt. Die Dichtscheibe mit ihrem radialen Fortsatz und dem Filmscharnier läßt sich auch aus einem federnden Kunststoff, insbesondere einem Polyacetatharz (POM), herstellen.

Nachfolgend wird die Erfindung anhand von Ausführungsbeispielen in Verbindung mit der Zeichnung beschrieben. Es zeigen:

Fig. 1 schematisch und im Schnitt den Hals- und Kopfbereich eines Patienten mit einem Tracheostoma, in das ein Ventilverschluß nach einem Ausführungsbeispiel der Erfindung eingesetzt ist;

Fig. 2 die Vorderansicht eines zusammengesetzten Verschlusses nach der Erfindung;

Fig. 3 die teilweise geschnittene Seitenansicht des Verschlusses nach Fig. 2;

Fig. 4 eine geschnittene Seitenansicht des Verschlusses nach Fig. 2 und 3 bei abgenommenem Flanschring und Ventilkörper;

Fig. 5 eine geschnittene Seitenansicht des Ventilkörpers mit eingesetzter Ventilklappe für den Verschluß nach Fig. 2 bis 4;

Fig. 6 eine vergrößerte Ansicht der Ventilklappe für den Verschluß nach Fig. 2 bis 5;

Fig. 7 eine Ansicht des Flanschrings für den Verschluß nach Fig. 2 bis 5;

Fig. 8 einen abgewandelten Flanschring für den Verschluß nach Fig. 2 bis 5;

Fig. 9 die Seitenansicht eines weiteren Ausführungsbeispiels für einen Verschluß nach der Erfindung;

Fig. 10 einen vergrößerten Ausschnitt des Verschlusses nach Fig. 9 im Bereich des Ventilkörpers;

Fig. 11 eine vergrößerte Vorderansicht der Ventilklappe für den Verschluß nach Fig. 9 und 10;

Fig. 12 eine vergrößerte Seitenansicht der Ventilklappe nach Fig. 11 im abgewinkelten Zustand;

Fig. 13 eine perspektivische Ansicht des Verschlusses nach Fig. 9 und 10;

Fig. 14 eine perspektivische Ansicht des Verschlusses nach Fig. 2 bis 5 mit abgewandeltem Abschlußorgan.

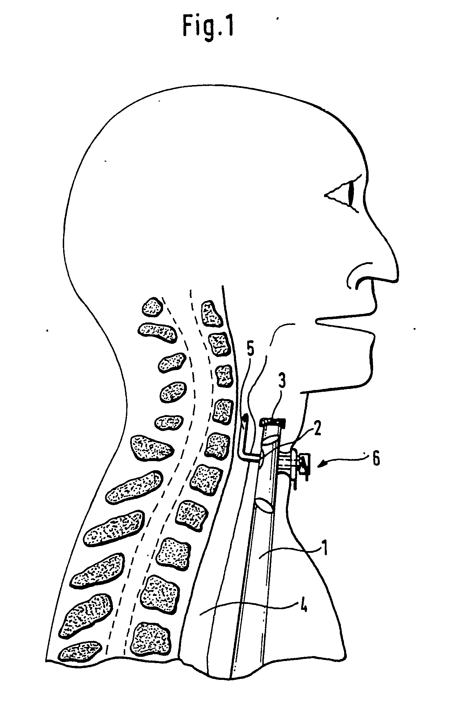

In Fig. 1 ist schematisch gezeigt, wie ein Tracheostoma-Verschluß nach der Erfindung angeordnet ist. Nach Totaloperation des Kehlkopfs steht der Luftweg (Trachea) 1 über eine unter Verwendung einer Schablone geschnittene, vorder Offnung und ein Stoma 2 mit der Außenluft in Verbindung. Ein Deckel 3 aus Muskelgewebe schließt die Trachea am abgesetzten Ende ab. Durch eine operativ hergestellte Verbindung zwischen dem Luftweg 1 und dem Speiseweg 4 führt eine sogenannte Sprech- oder Stimmprothese 5 in den Speiseweg, die das Sprechen auch ohne Kehlkopf ermöglicht. Solche Prothesen sind bekannt. Damit nun der Patient unter Ausatmen von Luft über die Sprechprothese 5 sprechen kann, muß das Stoma 2 verschlossen sein. Das geschieht entweder durch Auflegen eines Fingers oder mittels eines Ventilverschlusses 6. Ausführungsbeispiele für Ventilverschlüsse 6 nach der *Erfindung werden nachfolgend näher beschrieben.*

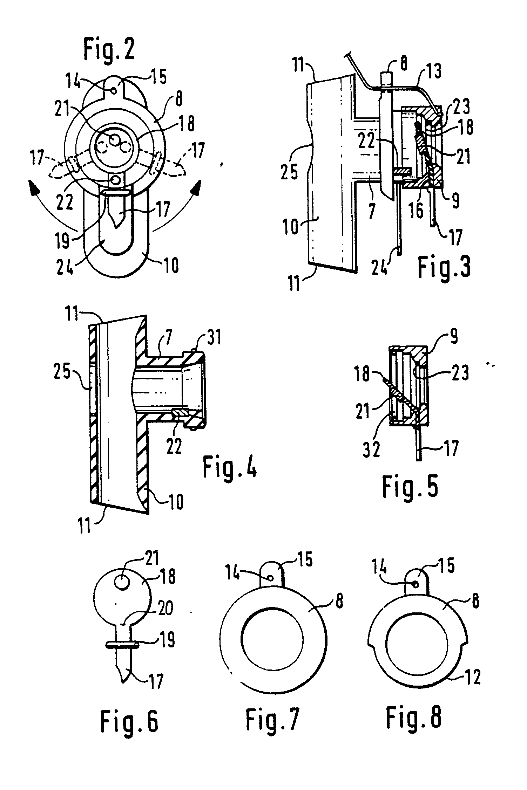

Bei dem Ausführungsbeispiel nach Fig. 2 bis 7 ist ein Kanülenstummel 7 mit einem aufgeschobenen Flanschring 8 und einem topfförmigen Ventilgehäuse 9 vorgesehen. An das rückwärtige (proximale) Ende des Kanülenstummels 7 schließt sich ein Rohrstück 10 mit schrägen Endflächen 11 an. Wie Fig. 1 zeigt, liegt das Rohrstück 10 später in dem Luftweg 1, und der Kanülenstummel 7 führt durch das Stoma 2 nach außen. Das Rohrstück 10 kann, da der Verschluß insgesamt aus weichelastischem Material, beispielsweise Silicongummi, mit einer für medizinische Zwecke zugelassenen Qualität hergestellt ist, durch das Stoma 2 auch nachträglich in den Luftweg 1 eingesetzt werden. Es besteht aber auch die Möglichkeit, den Verschluß noch während der Operation als Platzhalter einzubringen. Der verschiebbare Flanschring 8 wird an die Haut des Patienten dichtend angedrückt. Dabei kann im unteren Bereich des Flanschrings 8 eine Ausnehmung 12 (Fig. 8) vorgesehen sein, die eine bessere Anpassung an die anatomischen Verhältnisse im unteren Bereich des Stoma 2 ermöglicht.

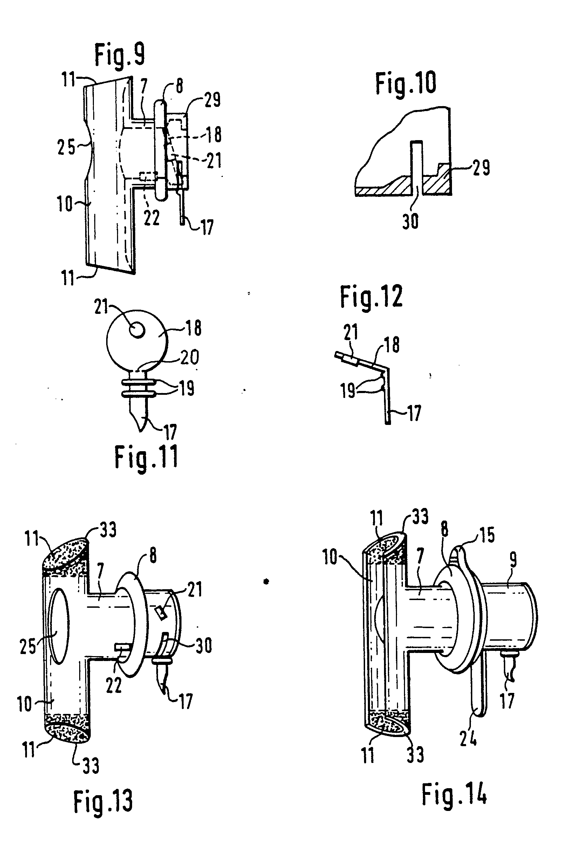

Das auf den Kanülenstummel 7 aufgesetzte, drehbare Ventilgehäuse 9 ist über ein Band 13, das zu einer Bohrung 14 (Fig. 7 und 8) in einem radialen Lappen 15 führt, mit dem Flanschring 8 verbunden, so daß es auch nach dem Abziehen vom Kanülenstummel 7 nicht verloren gehen kann. Statt dessen kann das Band 13 auch mit einem radialen Fortsatz 24 des Kanülenstummels 7 verbunden sein (nicht gezeigt), so daß der Flanschring 8 freibleibt und leichter ausgewechselt werden kann. Wenn der Flanschring 8 auf beiden Seiten symmetrisch abgerundet ist (vgl. Fig. 9), so kan er beliebig in beiden Richtungen auf den Kanülenstummel aufgesetzt werden. Ein leichteres Drehen des Ventilgehäuses 9 auf dem Kanülenstummel 7 erreicht man mittels eines umlaufenden Steges 31 im vorderen Bereich des Kanülenstummels. Das Ventilgehäuse 9 gleitet dann im wesentlichen auf diesem Steg 31. Ein entsprechender Steg 32 auf der Innenseite des Ventilgehäuses 9 schnappt nach dem Aufstecken über den Steg 31 und verhindert ein unbeabsichtigtes Abfallen.

Im vorderen, unteren Bereich weist das Ventilgehäuse 9 einen Schlitz 16 auf, durch den der radiale Fortsatz 17 einer scheibenförmigen Ventilklappe 18 (vgl. auch Fig. 6) führt. Der radiale Fortsatz 17 weist einen Anschlag in Form eines Stegs 19 auf, der durch den Schlitz 16 gezogen wird, bis er gerade austritt und an der unteren Wand des Ventilgehäuses 9 anliegt. Die Ventilklappe 18 hat dann die richtige Lage im Ventilgehäuse 9. Das "Scharnier" der Ventilklappe 18 ist ein sogenanntes Filmscharnier in Form einer Materialverdünnung 20 am Übergang des Fortsatzes 17 an die scheibenförmige Ventilklappe 18.

Die Ventilklappe 18 trägt im oberen Bereich einen kleinen und flachen Magneten 21, der beispielsweise in einer dünnhäutigen Tasche (nicht gezeigt) liegt und mit Kunststoff abgedeckt ist. In *die Innenwand des Kanülenstummels 7 ist ein* weiterer Magnet 22 (Fig. 4) eingebettet. Wenn sich das Ventilgehäuse 9 in der in Fig. 2 voll ausgezogenen Winkellage befindet und die Ventilklappe beim Einatmen nach unten gedrängt wird, entsteht eine magnetische Klebewirkung zwischen den beiden Magneten 21 und 22. Dabei braucht nur einer der beiden Magneten ein Permanentmagnet zu sein. Der jeweils andere kann aus einem einfachen Eisenstück mit entsprechendem Korrosionsschutz bestehen. Die magnetische Klebkraft zwischen den Magneten 21 und 22 ist so bemessen, daß erst bei einer verhältnismäßig hohen Strömungsgeschwindigkeit beim Ausatmen die Klappe 18 mit ihrem Magneten 21 vom Magneten 22 abgehoben wird und unter Erfüllung ihrer Rückschlagfunktion sich abdichtend gegen ihren Sitz 23 legt. Der Patient kann dieses Schließen des Ventils willkürlich dadurch herbeiführen, daß er kurz in Form eines Stoßes ausatmet. Nach dem Schließen der Klappe 18 kann der Patient dann mit Hilfe der Stimmprothese 5 sprechen.

Zur Einstellung der magnetischen Haltekraft zwischen den Magneten 21 und 22 läßt sich das Ventilgehäuse 9, zweckmäßig durch Anfassen eines Handgriffs 24, in einem in Fig. 2 angedeuteten Winkelbereich von beispielsweise je 50° nach beiden Seiten verdrehen. Die beiden Magneten 21, 22 liegen dann im offenen Zustand der Ventilklappe 18 nicht mehr vollflächig aufeinander, wodurch die Haltekraft verringert wird. Entsprechend kleiner wird die Ausströmgeschwindigkeit, bei der die Klappe 18 schließt.

Da immer sichergestellt sein muß, daß das Ventil bei heftigem Ausatmen, beispielsweise beim Husten, öffnet, ist die Ventilklappe 18 aus weichelastischem Material hergestellt und so bemessen, daß sie beim Auftreten eines gewissen Staudrucks ihren Sitz 23 überwindet und aus dem Ventilgehäuse 9 nach vorn austritt. Statt dessen kann aber das Ventilgehäuse 9 so ausgebildet sein, daß es beim Husten automatisch abgedrückt wird.

Anstelle der magnetischen Haltekraft zwischen den Magneten 21 und 22 kann die Ventilklappe 18 auch nur mit einem Zusatzgewicht ausgestattet sein, das beispielsweise ähnliche Form wie der Magnet 21 hat. Das Gewicht und die Scharnierausbildung müssen dann so getroffen sein, daß die Klappe 18 im Ruhezustand, also ohne hindurchströmende Luft, am Boden des Kanülenstummels aufliegt und erst bei höheren Ausatmungsgeschwindigkeiten schließt. Auch hier führt eine Verdrehung des Ventilgehäuses 9 zu einer Einstellung für die Luftgeschwindigkeit, bei der das Schliessen erfolgt.

Das Rohrstück 10 weist in seiner dem Kanülenstummel 7 gegenüberliegenden Wand eine Öffnung 25 auf, durch die die Stimmprothese 5 (vgl. Fig. 1) in den Verschluß 6 führt. Anstelle der Öffnung 25 kann das Rohrstück 10 auch vollständig geöffnet sein, wie die perspektivische Ansicht in Fig. 14 zeigt.

Das Ausführungsbeispiel nach Fig. 9 unterscheidet sich von dem vorstehend beschriebenen Ausführungsbeispiel dadurch, daß das Ventilgehäuse 29 nunmehr einstückig an den Kanülenstummel 7 angeformt ist. Im übrigen entspricht das Ventil mit seiner Klappe 18 und den Magneten 21, 22 dem Ausführungsbeispiel nach Fig. 2 bis 5. Entsprechendes gilt für den Flanschring 8 und das Rohrstück 10.

Ähnlich wie beim Ausführungsbeispiel nach Fig. 2 bis 5 ist die Ventilklappe 18 mit ihrem radialen Fortsatz 17 dadurch im Ventilgehäuse 29 befestigt, daß der Fortsatz 17 durch einen Schlitz 30 gesteckt ist. Dabei weist der radiale Fortsatz in Abwandlung des Ausführungsbeispiels nach Fig. 6 zwei Stege 19 als Anschläge auf, von denen nach dem Einstecken einer außerhalb und der andere innerhalb des Ventilgehäuses 29 liegt. Der Schlitz 30 ist, wie der vergrößerte Ausschnitt gemäß Fig. 10 zeigt, in Umfangsrichtung so verbreitert, daß der radiale Fortsatz 17 im Schlitz 30 in Umfangsrichtung verschoben werden kann. Damit läßt sich die gleiche Anpassung wie beim Verdrehen des Ventilgehäuses 9 beim Ausführungsbeispiel gemäß Fig. 2 bis 5 erzielen. Der Schlitz 30 ist abweichend von der Darstellung in Fig. 10 so gestaltet, daß seine Ränderdort, wo sich der Fortsätz 19 nicht befindet, dichtend aneinanderlegen.

Fig. 12 zeigt die Ventilklappe 18 nach Fig. 1 in der Seitenansicht. Dabei ist in Fig. 12 die Ruhelage dargestellt, also diejenige Lage, in der die Klappe 18 mit ihrem Fortsatz 17 gefertigt worden ist. Dadurch läßt sich zusätzlich über das Scharnier 20 eine Federkraft erzeugen, die die Ventilscheibe 18 in die Ruhelage gemäß Fig. 12 drängt.

Fig. 13 zeigt das Ausführungsbeispiel gemäß Fig. 9 noch einmal perspektivisch. Anstelle des Rohrstücks 10 mit Öffnung 25 kann auch hier das rinnenförmig aufgeschnittene Rohrstück 10 gemäß Fig. 14 verwendet werden. In den strichpunktierten Endbereichen 33 wird das Rohrstück 10 mit Vorteil aus weich eingestelltem Material hergestellt, so daß Irritationen oder gar Verletzungen des Patienten vermieden werden.

Während schon beim Ausführungsbeispiel nach Fig. 2 bis 5 die Bauhöhe des Verschlusses und insbesondere dasjenige Maß, mit dem der Verschluß aus dem Stoma hervorsteht, gegenüber dem Stand der Technik wesentlich verringert worden ist, ermöglicht das Ausführungsbeispiel nach Fig. 9 durch das integrierte Ventilgehäuse 29 eine weitere Verbesserung in dieser Richtung. Aus ästhetischen Gründen ist eine solche Verringerung der Bauhöhe für die Patienten besonders wichtig.

**Patentansprüche**

1. Tracheostoma-Verschluß mit einem zur Ermöglichung des Sprechens vom Patienten beim Ausatmen willkürlich betätigbaren Ventil, einem in das Stoma (2) einführbaren Kanülenstummel (7), der ein in die Trachea (1) einbringbares Abschlußorgan (10) besitzt, einem auf den Kanülenstummel (7) klemmend oder rastend aufschiebbaren oder mit dem Kanülenstummel fest verbundenen Flanschring (8), einem Ventilgehäuse (9, 29), das den Kanülenstummel (7) nach außen abschließt, und einer Vorrichtung, die bei Überschreiten eines vorgegebenen Staudrucks das Ventil unwirksam macht, wobei im Ventilgehäuse (9, 29) eine Rückschlag-Ventilkappe (18) schwenkbar gelagert ist, die in Einatmungsrichtung öffnet und in Ausatmungsrichtung schließt, wenn eine vorgegebene Strömungsgeschwindigkeit überschritten wird, dadurch gekennzeichnet daß die Ventilklappe (18) ein magnetisch wirksames Bauteil (21) aufweist, das mit einem weiteren magnetisch wirksamen Bauteil (22) am oder in der Wand des Ventilgehäuses (9, 29) zusammenwirkt und die Ventilklappe (18) im geöffneten Zustand hält, und daß wenigstens eines der magnetisch wirksamen Bauteile ein Permanentmagnet ist.

2. Tracheostoma-Verschluß mit einem zur Ermöglichung des Sprechens vom Patienten beim Ausatmen willkürlich betätigbaren Ventil, einem in das Stoma (2) einführbaren Kanülenstummel (7), der ein in die Trachea (1) einbringbares Abschlußorgan (10) besitzt, einem auf den Kanülenstummel (7) klemmend oder rastend aufschiebbaren oder mit dem Kanülenstummel fest verbundenen Flanschring (8), einem Ventilgehäuse (9, 29), das den Kanülenstummel (7) nach außen abschließt, und einer Vorrichtung, die bei Überschreiten eines vorgegebenen Staudrucks das Ventil unwirksam macht, wobei im Ventilgehäuse (9, 29) eine Rückschlag-Ventilkappe (18) schwenkbar gelagert ist, die in Einatmungsrichtung öffnet und in Ausatmungsrichtung schließt, wenn eine vorgegebene Strömungsgeschwindigkeit überschritten wird, dadurch gekennzeichnet, daß die Ventilklappe (18) im in der Betriebslage unteren Bereich des Ventilgehäuses (9, 29) gelagert ist und durch ein Zusatzgewicht (21) in der geöffneten Lage gehalten wird.

3. Verschluß nach Anspruch 1 oder 2, dadurch gekennzeichnet, daß die Ventilklappe (18) und/oder ihr Sitz (23) aus nachgiebigem Material besteht, derart, daß die Klappe (18) nach dem Schließen durch ihren Sitz (23) hindurchtreten kann, wenn der vorgegebene Staudruck überschritten wird.

4. Verschluß nach einem der Ansprüche 1 bis 3, dadurch gekennzeichnet, daß die Ventilklappe (18) durch Federkraft im geöffneten Zustand gehalten wird.

5. Verschluß nach einem der Ansprüche 1 bis 4, dadurch gekennzeichnet, daß die Kraft, mit der die Ventilklappe (18) im geöffneten Zustand gehalten wird, einstellbar ist.

6. Verschluß nach Anspruch 5, dadurch gekennzeichnet, daß die Lagerstelle der Ventilklappe (18) in Umfangsrichtung des Kanülenstummels (7) verschiebbar ist.

7. Verschluß nach einem der Ansprüche 1 bis 6, dadurch gekennzeichnet, daß das Ventilgehäuse (9) auf den Kanülenstummel (7) aufgesteckt und gegen diesen verdrehbar ist.

8. Verschluß nach Anspruch 7, dadurch gekennzeichnet, daß der Kanülenstummel (7) im Bereich seiner Berührungsfläche mit dem Ventilgehäuse (9) einen umlaufenden Steg (31) aufweist.

9. Verschluß nach Anspruch 8, dadurch gekennzeichnet, daß das Ventilgehäuse (9) im Bereich seiner Berührungsfläche mit dem Kanülenstummel (7) einen umlaufenden Steg (32) besitzt.

10. Verschluß nach einem der Ansprüche 1 bis 6, dadurch gekennzeichnet daß das Ventilgehäuse (29) Teil des Kanülenstummels (7) ist.

11. Verschluß nach einem der Ansprüche 1 bis 10, dadurch gekennzeichnet, daß die Ventilklappe die Form einer dünnen Scheibe (18) hat und über ein Filmscharnier (20) mit einem radialen Fortsatz (17) verbunden ist, der abdichtend durch einen Schlitz (16, 30) in der Wand des Ventilgehäuses (9, 29) gesteckt ist.

12. Verschluß nach Anspruch 11, dadurch gekennzeichnet, daß der radiale Fortsatz wenigstens einen Anschlag (19) aufweist, der die Einstecktiefe definiert und begrenzt.

13. Verschluß nach Anspruch 11 und 12 oder 13, dadurch gekennzeichnet, daß der Schlitz (30) in Umfangsrichtung des Ventilgehäuses (29) so breit ist, daß der Fortsatz (17) der Ventilklappe (18) in seiner Winkellage verstellbar ist.

14. Verschluß nach einem der Ansprüche 1 bis 13, dadurch gekennzeichnet, daß der Flanschring (8) auf einem Teil seines Umfangs eine Ausnehmung (Fig. 8: 12) aufweist.

15. Verschluß nach einem der Ansprüche 1 bis 14, dadurch gekennzeichnet, daß der Flanschring (8) mit einer eine Bohrung (14) aufweisenden, radial abstehenden Lasche (15) versehen ist und daß in der Bohrung ein mit dem Ventilgehäuse (9) verbundener Haltefaden (13) festgelegt ist.

16. Verschluß nach einem der Ansprüche 1 bis 14, dadurch gekennzeichnet, daß der Kanülenstummel (7) eine radial abstehende Lasche (24) aufweist und daß das Ventilgehäuse (9) über einen Haltefaden mit der Lasche verbunden ist.

17. Verschluß nach einem der Ansprüche 1 bis 16, dadurch gekennzeichnet, daß das Abschlußorgan eine an das innere Ende des Kanülenstummels (7) angesetztes, gegen den Kanülenstummel (7) offenes Rohrstück (10) ist.

18. Verschluß nach Anspruch 17, dadurch gekennzeichnet, daß die Enden des Rohrstücks (10) schräg (11) abgesetzt sind, derart, daß das Rohrstück (10) an der dem Kanülenstummel (7) entgegengesetzten Seite kürzer ist.

19. Verschluß nach Anspruch 17 oder 18, dadurch gekennzeichnet, daß das Rohrstück (10) an der dem Kanülenstummel gegenüberliegenden Wand eine Öffnung (25) aufweist.

20. Verschluß nach einem der Ansprüche 17 bis 19, dadurch gekennzeichnet, daß das Rohrstück (10) auf der dem Kanülenstummel (7) gegenüberliegenden Seite vollständig geöffnet ist.

21. Verschluß nach einem der Ansprüche 17 bis 20, dadurch gekennzeichnet, daß das Rohrstück (10) im Bereich seiner Enden aus weich eingestelltem Material hergestellt ist.

22. Verschluß nach einem der Ansprüche 1 bis 21, gekennzeichnet durch seine Herstellung aus Silicongummi.

23. Verschluß nach einem der Ansprüche 1 bis 22, dadurch gekennzeichnet, daß die Ventilscheibe (18) mit ihrem radialen Fortsatz (17) und dem Filmscharnier (20) aus einem federnden Kunststoff, insbesondere einem Polyacetatharz (POM), hergestellt ist.

**Revendications**

1. Obturateur pour trachéotomie, comportant une valve pouvant être actionnée intentionnellement pour permettre à des patients de parler en expirant, un tronçon de canule (7) pouvant être introduit dans l'ouverture (2), qui comporte un élément d'obturation (10) pouvant être mis en place dans la trachée (1), un collier de bride (8) coulissant sur le tronçon de canule (7) de manière à se bloquer ou à s'encliqueter ou fixé solidement au tronçon de canule (7), un boîtier de valve (9, 29) qui ferme le tronçon de canule (7) vers l'extérieur et un dispositif qui supprime l'action de la valve lorsqu'une pression dynamique prédéterminée est dépassée, dans lequel un clapet de valve (18) de non-retour est logé dans le boîtier de valve (9, 29) de manière à pouvoir pivoter, ledit clapet s'ouvrant dans le sens de l'inspiration et se fermant dans le sens de l'expiration si une vitesse d'écoulement prédéterminée est dépassée, caractérisé en ce que le clapet de valve (18) comporte un élément (21) à action magnétique qui coopère sur ou dans la paroi du boîtier de valve (9, 29) avec un autre élément (22) à action magnétique et maintient le clapet de valve (18) en position ouverte, et en ce qu'au moins un des éléments à action magnétique est un aimant permanent.

2. Obturateur pour trachéotomie, comportant une valve pouvant être actionnée intentionnellement pour permettre à des patients de parler en expirant, un tronçon de canule (7) pouvant être introduit dans l'ouverture (2), qui comporte un élément d'obturation (10) pouvant être mis en place dans la trachée (1), un collier de bride (8) coulissant sur le tronçon de canule (7) de manière à se bloquer ou à s'encliqueter ou fixé solidement au tronçon de canule (7), un boîtier de valve (9, 29) qui ferme le tronçon de canule (7) vers l'extérieur et un dispositif qui supprime l'action de la valve lorsqu'une pression dynamique prédéterminée est dépassée, dans lequel un clapet de valve (18) de non-retour est logé dans le boîtier de valve (9, 29) de manière à pouvoir pivoter, ledit clapet s'ouvrant dans le sens de l'inspiration et se fermant dans le sens de l'expiration si une vitesse d'écoulement prédéterminée est dépassée, caractérisé en ce que le clapet de valve (18) est logé, en position de fonctionnement, dans la partie inférieure du boîtier de valve (9, 29) et est maintenu en position ouverte par un poids supplémentaire (21).

3. Obturateur selon la revendication 1 ou 2, caractérisé en ce que le clapet de valve et/ou son siège (23) se composent d'un matériau souple de manière à ce que le clapet (18) puisse passer à travers son siège (23) après l'obturation si la pression dynamique prédéterminée est dépassée.

4. Obturateur selon l'une quelconque des revendications 1 à 3, caractérisé en ce que le clapet de valve (18) est maintenu en position ouverte par un effet de ressort.

5. Obturateur selon l'une quelconque des revendications 1 à 4, caractérisé en ce que la force par laquelle le clapet de valve (18) est maintenu en position ouverte peut être réglée.

6. Obturateur selon la revendication 5, caractérisé en ce que le point d'appui du clapet de valve (18) peut être déplacé dans le sens périphérique du tronçon de canule (7).

7. Obturateur selon l'une quelconque des revendications 1 à 6, caractérisé en ce que le boîtier de valve (9) est enfilé sur le tronçon de canule (7) et peut être tourné par rapport à celui-ci.

8. Obturateur selon la revendication 7, caractérisé en ce que le tronçon de canule (7) présente au niveau de sa surface de contact avec le boîtier de valve (9) une barrette périphérique (31).

9. Obturateur selon la revendication 8, caractérisé en ce que le boîtier de valve (9) présente au niveau de sa surface de contact avec le tronçon de canule (7) une barrette périphérique (32).

10. Obturateur selon l'une quelconque des revendications 1 à 6, caractérisé en ce que le boîtier de valve (29) est une partie du tronçon de canule (7).

11. Obturateur selon l'une quelconque des revendications 1 à 10, caractérisé en ce que le clapet de valve a la forme d'un disque mince (18) et est relié à un prolongement radial (17) au moyen d'une charnière pelliculaire (20), lequel prolongement radial (17) est introduit de manière étanche dans une fente (16, 30) située dans la paroi du boîtier de valve (9, 29).

12. Obturateur selon la revendication 11, caractérisé en ce que le prolongement radial (17) présente au moins une butée (19) qui définit et délimite la profondeur d'insertion.

13. Obturateur selon les revendications 10 et 11 ou 12, caractérisé en ce que la fente (30) est, dans la direction périphérique du boîtier de valve (29), d'une largeur telle, que le prolongement (17) du clapet de valve (18) peut être déplacé dans sa position relative.

14. Obturateur selon l'une quelconque des revendications 1 à 13, caractérisé en ce que le collier de bride (8) présente un renfoncement sur une partie de sa périphérie (figure 8, réf. 12).

15. Obturateur selon l'une quelconque des revendications 1 à 14, caractérisé en ce que le collier de bride (8) est pourvu d'une languette (15) décalée radialement et présentant un perçage (14) et en ce qu'un fil de retenue (13) relié au boîtier de valve (9) est attaché au moyen dudit perçage.

16. Obturateur selon l'une quelconque des revendications 1 à 14, caractérisé en ce que le tronçon de canule (7) présente une languette (24) décalée radialement et en ce que le boîtier de valve (9) est relié à la languette au moyen d'un fil de retenue.

17. Obturateur selon l'une quelconque des revendications 1 à 16, caractérisé en ce que l'élément d'obturation est un élément de tube (10) abouté à l'extrémité interne du tronçon de canule (7) et qui est ouvert par rapport au tronçon de canule (7).

18. Obturateur selon la revendication 17, caractérisé en ce que les extrémités (11) de l'élément de tube (10) sont décalées en biseau de manière telle, que l'élément de tube (10) est plus court sur son côté opposé au tronçon de canule (7).

19. Obturateur selon la revendication 17 ou 18, caractérisé en ce que l'élément de tube (10) présente une ouverture (25) dans la paroi située vis-à-vis du tronçon de canule (7).

20. Obturateur selon l'une quelconque des revendications 17 à 19, caractérisé en ce que l'élément de tube (10) est entièrement ouvert sur le côté situé vis-à-vis du tronçon de canule (7).

21. Obturateur selon l'une quelconque des revendications 17 à 20, caractérisé en ce que l'élément de tube (10) est fabriqué, au niveau de ses extrémités, à partir d'un matériau souple.

22. Obturateur selon l'une quelconque des revendications 1 à 21, caractérisé en ce qu'il est fabriqué en caoutchouc silicone.

23. Obturateur selon l'une quelconque des revendications 1 à 22, caractérisé en ce que le disque de valve (18), ainsi que son prolongement radial (17) et la charnière pelliculaire (20) sont fabriqués en matière plastique élastique, en particulier une résine de polyacétate (POM).

**Claims**

1. A tracheal fistula closure having: a valve adapted to be activated at will to enable the patient to speak during exhalation; a cannular spigot (7) which is introducible into the fistula (2) and which has a closure member (10) introducible into the trachea (1); a flanged ring (8) adapted to be pushed on to the cannular spigot (7) in clamping or latching engagement therewith or to be rigidly connected to the cannular spigot; a valve body (9, 29) which seals the cannular spigot (7) off from the outside; and means which render the valve inoperative upon a predetermined dynamic pressure being exceeded, a check valve flap (18) being pivotally mounted in the valve body (9, 29) and opening in the inhalation direction and closing in the exhalation direction when a predetermined flow velocity is exceeded, characterised in that the valve flap (18) has a magnetically operative component (21) which co-operates with another magnetically operative component (22) on or in the valve body wall and which keeps the valve flap (18) open, and at least one of the magnetically operative components is a permanent magnet.

2. A closure having: a valve adapted to be activated at will to enable the patient to speak during exhalation; a cannular spigot (7) which is introducible into the fistula (2) and which has a closure member (10) introducible into the trachea (1); a flanged ring (8) adapted to be pushed on to the cannular spigot (7) in clamping or latching engagement therewith or to be rigidly connected to the cannular spigot; a valve body (9, 29) which seals the cannular spigot (7) off from the outside; and means which render the valve inoperative upon a predetermined dynamic pressure being exceeded, a check valve flap (18) being pivotally mounted in the valve body and opening in the inhalation direction and closing in the exhalation direction when a predetermined flow velocity is exceeded, characterised in that the valve flap (18) is mounted in that part of the valve body (9, 29) which is at the bottom in the operative position, the valve flap (18) being retained in the open position by an additional weight (21).

3. A closure according to claim 1 or 2, characterised in that the valve flap (18) and/or its seat are and/or is made of a resilient material so that the flap (18) can after closure pass through its seat (23) when the predetermined dynamic pressure is exceeded.

4. A closure according to any of claims 1-3, characterised in that the valve flap (18) is kept open by spring force.

5. A closure according to any of claims 1-4, characterised in that the force with which the valve flap (18) is kept open is adjustable.

6. A closure according to claim 5, characterised in that the bearing position of the valve flap (18) is adjustable in the peripheral direction of the cannular spigot (7).

7. A closure according to any of claims 1-6, characterised in that the valve body (9) is pushed on to the cannular spigot (7) and is rotatable relatively thereto.

8. A closure according to claim 7, characterised in that the cannular spigot (7) has near its contact surface with the valve body (9) a peripheral web (31).

9. A closure according to claim 8, characterised in that the valve body (9) has near its contact surface with the cannular spigot (7) a peripheral web (32).

10. A closure according to any of claims 1-6, characterised in that the valve body (29) is part of the cannular spigot (7).

11. A closure according to any of claims 1-10, characterised in that the valve flap is in the form of a thin disc (18) and is connected by way of a film-like hinge (20) to a radial prolongation (17) pushed sealingly through a slot (16, 30) in the valve body wall.

12. A closure according to claim 11, characterised in that the radial prolongation has at least one abutment (19) which defines and limits the depth of insertion.

13. A closure according to claims 11 and 12 or 13, characterised in that the slot (30) is so wide in the peripheral direction of the valve body (29) that the valve flap prolongation (17) is adjustable in angular position.

14. A closure according to any of claims 1-13, characterised in that the flanged ring (8) is formed with a recess (Fig. 8: 12) over some of its periphery.

15. A closure according to any of claims 1-14, characterised in that the flange ring (8) has a radially projecting tab (15) which is formed with a bore (14), and a retaining thread (13) connected to the valve body (9) is secured in the latter bore (14).

16. A closure according to any of claims 1-14, characterised in that the cannular spigot (7) has a radially projecting tab (24) and the valve body (9) is connected thereto by way of a retaining thread.

17. A closure according to any of claims 1-16, characterised in that the closure element is a tubular member (10) which is disposed on the inner end of the cannular spigot (7) and which is open theretowards.

18. A closure according to claim 17, characterised in that the ends of the tubular member (10) have a bevelled offset so that the tubular member (10) is shorter on the side remote from the cannular spigot (7).

19. A closure according to claim 17 or 18, characterised in that the tubular member (10) is formed with an aperture (25) in the wall remote from the cannular spigot (7).

20. A closure according to any of claims 17-19, characterised in that the tubular member (10) is completely open on the side remote from the cannular spigot (7).

21. A closure according to any of claims 17-20, characterised in that the tubular member (10) is made of a soft material near its ends.

22. A closure according to any of claims 1-21, characterised in that it is made of silicone rubber.

23. A closure according to any of claims 1-22, characterised in that the valve disc (18), its radial prolongation (17) and the film-like hinge (20) are made of a resilient plastics, more particularly a polyacetate resin (POM).

## Fig.1

Fig.2

Fig.3

Fig.4

Fig.5

Fig.6

Fig.7

Fig.8

Fig.9

Fig.10

Fig.12

Fig.11

Fig.13

Fig.14